# EUROPEAN PATENT APPLICATION

(11) **EP 4 620 491 A1**
(43) Date of publication of application: **24.09.2025**
(21) Application number: 24165054.8
(22) Date of filing: 21.03.2024
(51) Int. Cl.: A61L 24/00, A61L 24/02, A61L 27/12, A61L 27/50

(54) **COMPRESSED TABLET OF CALCIUM PHOSPHATE**

(71) Applicant: Biomimetic Innovations Limited, V14 RW92 Co Clare (IE)
(72) Inventor: PROCTER, Philip, 01220 Divonne les Bains (FR); ENGQVIST, Håkan, 752 29 Uppsala (SE); INSLEY, Gerard, Rathkeale, Co. Limerick, - (IE); DORRELL, Paul, Castleconnell, Co. Limerick, - (IE)
(74) Representative: Brann AB

(57) **Abstract**

A solid object having a shape wherein the solid object comprises a powder wherein the powder is a mixture of particles wherein said particles comprises a calcium phosphate compound and phosphoserine or phosphocreatine; and wherein the solid object has a density of 1.20-1.80g/cm³.

## Description

### FIELD OF INVENTION

The present invention relates to a solid object comprising a compressed powder, a method of preparing the same, a kit comprising the solid object and a method of treating a patient using the solid object.

### BACKGROUND

Calcium phosphates (CaP) is a mineral that is widely used in medical applications due to its similarity to the mineral components of bone and teeth and its biocompatibility.

The field of biomaterials includes fixation of implants to tissues as well as tissue repair and void filling. The limited mechanical strength of implants in combination with adhesives has remained an issue within the field of implants and biomaterials.

US2012288446 (US'446) discloses an adhesive comprising a powder composition of a multivalent metal compound, a compound comprising a phosphoserine oligomer or a phosphoserine capped polymer wherein the latter compound is present at 10-90wt%. US'446 discloses experimental data using tetra calcium phosphate (TTCP) as the multivalent metal compound and phosphoserine-ethyleneglycol-diglycidyl-phosphoserine for example and obtains, when mixed with water, an adhesive strength of up to 3.76MPa when adhered to bone.

US20130122057 (US'057) discloses a bone restorative powder composition comprising amino acid phosphate species, a multivalent metal compound and a bioactive glass material containing ionic functional groups. US'057 discloses examples using a composing comprising TTCP as the multivalent metal compound and phosphoserine together with various amounts of Combeite Bioactive glass and water and adhere it to bone. The shear strengths obtained varied between 0.75-2. 13MPa.

US8765189 (US'189) teaches an adhesive powder composition comprising a multivalent metal compound and a phosphoserine like compound in an amount of 10-90wt%. US'189 discloses an adhesion shear strength to cortical bone after 5 minutes of 130-890kPa when using TTCP as the multivalent metal compound, various phosphorylated compounds and mixing it with water and 650kPa when using α-TCP and phosphoserine.

However, calcium phosphate-based compositions are powders which means that they are hard to handle for a practitioner, require containers to hold the powders and require mixing manually or via a mixing system.

### SUMMARY OF INVENTION

The object of the present invention is to overcome the drawbacks of the prior art by providing a calcium phosphate composition in a format that is easy to handle and that facilitates fast and efficient mixing with minimal or no manual or machine input.

This invention aims to also overcome the risks associated with using injectable calcium phosphate materials, particularly in the spinal area. Current risks with these materials are associated with unwanted migration of the material and the potential for pressurisation leading to the potential for nerve damage and embolism. This invention allows for controlled, accurate placement of the calcium phosphate material with minimal pressure application.

In a first aspect the present invention relates to a solid object having a shape obtainable by compressing a powder at a pressure of 10 to 150 MPa and wherein the powder is a mixture of particles wherein said particles comprises a calcium phosphate compound and phosphoserine or phosphocreatine.

In a second aspect the present invention relates to a solid object having a shape wherein the solid object comprises a powder wherein the powder is a mixture of particles wherein said particles comprises a calcium phosphate compound and phosphoserine or phosphocreatine; and wherein the solid object has a density of 1.20-1.80g/cm³.

In a third aspect the present invention relates to a method of preparing the solid object according to the present invention wherein the method comprises the steps of:
a. Providing a powder mixture comprising a calcium phosphate compound, phosphoserine and optionally a calcium silicate;
b. Arranging the powder in a mould having the shape; and
c. Compressing the powder mixture during a first period of time at a pressure of 10-150 MPa.

In a fourth aspect the present invention relates to a kit comprising at least one solid object according to any one of claim 1 to 11 and a syringe comprising a chamber and a plunger arranged in the chamber; wherein the syringe is configured to receive the solid object and an aqueous solution, and wherein the syringe is further configured to mix the aqueous solution with the solid object when pushing the plunger.

In a fifth aspect the present invention relates to a method of treating a patient comprising:
a. Providing the solid object according to the present invention wherein the solid object is in the shape of a tablet, a ring, a sphere, a disc, a cylinder, a tube, a rectangular or cubic block, sheet, a cone or a stack of rings;
b. Arranging the solid object at or near a site to be treated;
c. Adding an aqueous solution to the solid object or allowing body fluid to come into contact with the solid object; and
d. Inserting a screw or an implant into the solid object.

In a sixth aspect the present invention relates to a solid object obtainable by the method of preparing a solid object according to the present invention.

### EMBODIMENTS OF THE INVENTION

In one embodiment according to any one of the aspects the density is 1.30-1.70 g/cm³, preferably 1.40-1.65 g/cm³, more preferably 1.45-1.60 g/cm³.

In one embodiment according to any one of the aspects the shape is a tablet, a ring, a sphere, a disc, a cylinder, a tube, a rectangular or cubic block, sheet or a cone.

In one embodiment according to any one of the aspects the height of the solid object is 1 mm or more, preferably 2 mm or more, more preferably 3 mm or more, more preferably 4 mm or more, but preferably not more than 10 mm, preferably 8 mm or less, more preferably 7 mm or less, more preferably 6 mm or less.

In one embodiment according to any one of the aspects the particles have a mean particle size of 1 µm to 20 µm, preferably 3-15µm.

In one embodiment according to any one of the aspects the particles have a Dspan of 7 or lower, preferably 3 or lower, but preferably higher than 2.

In one embodiment according to any one of the aspects the calcium phosphate compound is selected from tetra calcium phosphate (TTCP) or α-tricalcium phosphate (α-TCP).

In one embodiment according to any one of the aspects the amount of calcium phosphate is 65-85wt%.

In one embodiment according to any one of the aspects the amount of phosphoserine or phosphocreatine is 15 to 35wt% of the total weight of the solid object, preferably 20 to 30wt%, more preferably 22 to 27wt%.

In one embodiment according to any one of the aspects the powder further comprises calcium silicate, preferably in an amount of 0.2-2wt%, preferably 0.5-1.5wt%.

In one embodiment according to any one of the aspects the calcium silicate is selected from a mono-, di- or tricalcium silicate or a mixture of di- and tricalcium silicate.

In one embodiment according to any one of the aspects the object is obtainable by compressing a powder at a pressure of 110-150MPa wherein the solid object has a diameter of about 2-4mm or a surface area of 3-12mm².

In one embodiment according to any one of the aspects the object is obtainable by compressing a powder at a pressure of 80-120MPa wherein the solid object has a diameter of about 3.5-5.5mm or a surface area of 9.5-24mm².

In one embodiment according to any one of the aspects the object is obtainable by compressing a powder at a pressure of 40-90MPa wherein the solid object has a diameter of about 5-8mm or a surface area of 19.5-50mm².

In one embodiment according to any one of the aspects the object is obtainable by compressing a powder at a pressure of 10-50MPa wherein the solid object has a diameter of about 8mm or larger or a surface area of 50mm² or larger.

In embodiment according to the third aspect the pressure is 110-150MPa when the solid object has a diameter of about 2-4mm or a surface area of 3-12mm².

In embodiment according to the third aspect the pressure is 80-120MPa when the solid object has a diameter of about 3.5-5.5mm or a surface area of 9.5-24mm².

In embodiment according to the third aspect the pressure is 40-90MPa when the solid object has a diameter of about 5-8mm or a surface area of 19.5-50mm².

In embodiment according to the third aspect the pressure is 10-50MPa when the solid object has a diameter of about 8mm or larger or a surface area of 50mm² or larger.

In embodiment according to the third aspect the first period of time is at least 10 seconds, preferably 30 to 120 seconds.

In one embodiment according to the fifth aspect the site to be treated is bone, preferably vertebral bodies of the spine

In one embodiment according to the fifth aspect wherein the site is a spine wherein the method comprises
a. Providing the solid object according to the present invention wherein the solid object is in the shape of a cylinder, a ring, a tube or a stack of rings;
b. Arranging the solid object at or near the site to be treated by preparing one or more holes in one or more vertebrate bodies in the spine, preferably in two adjacent vertebrate bodies, and arranging the solid object in each hole;
c. Adding an aqueous solution to each solid object or allowing body fluid to come into contact with the solid object;
d. Inserting a screw or an implant into each solid object; and e. Optionally connecting the screws or the implants via a rod.

All the embodiments presented herein relate to all the aspects of the present invention and may be combined unless stated otherwise.

### BRIEF DESCRIPTION OF THE FIGURES

Figure 1, illustrations of solid objects according to the present invention a) a circular tablet, b) a circular tablet, c) a stack of rings, d) a cylinder and e) a rectangular block.
Figure 2, illustration of a method of preparing a solid object according to the present invention using a die tool, a) loading of powder, b) compressing powder and c) ejecting solid object.
Figure 3, illustrations of treating a spine injury or defect according to the present invention, a) side view of spine, b) top view of spine and c) top view of spine.
Figure 4, illustration of forming of paste by adding aqueous solution to a tablet in a recess.
Figure 5, illustration of insertion of a screw into the paste and curing.
Figure 6, illustrations of adhering talus bone using tablets of the present invention (Fig. 6a-c), and illustrations of fixation of bone using a metal screw reinforcement implant comprising tablets of the present invention (Fig. 6d-f).

### DETAILED DESCRIPTION

In the present application the wording "aqueous solution" encompasses water and water of any purity, and body fluids e.g. blood. The water may be but is not limited to tap water, distilled water, filtered water, reverse osmosis water or deionized water. The aqueous solution may also be a buffer such as PBS or any suitable saline buffer.

The aqueous solution may contain a soluble reaction retardant. This retardant may be trisodium citrate or other compound that has an effect of delaying the chemical reaction between the powder component and aqueous solution.

In the present application particle size is determined using Particle Size Analysis equipment such as supplied by Bruker.

In the present application density is determined using calculation of the volume of a known mass of powder.

In the present application the term "cross section length" denotes the longest dimension of a 2-dimensional non-circular shape.

In the present application is the terms "solid object" and "solid body" denote the same thing and are used interchangeably.

In the present application, the term "surface area" means the area onto which the compression load is applied to when preparing the solid object.

### Solid object

The present inventors have surprisingly found that a compressed powder of CaP and phosphoserine or phosphocreatine and preferably calcium silicate forms, when mixed with an aqueous solution, a paste much easier in comparison with the uncompressed powder.

When mixing an aqueous solution with a powder of the present invention the mixing takes time partly due to the fact that the powder sticks to surfaces and requires mechanical mixing, and a powder is hard to handle. Instead, surprisingly the solid object of the present invention is easily soaked when water or an aqueous solution is added and by applying light pressure to the soaked solid body an adhesive paste is formed. Thereby, less energy is required to combine the powder components to form a paste in comparison with a powder where stirring and mixing is required, and it is also believed that the mechanical properties (e.g. compressive strength) of the obtained paste is better than when an uncompressed non-mechanically mixed powder is used.

This goes against what is common general knowledge regarding the mixing of powders where powders or finely divided parts are more easily mixed than solid objects.

A further advantage is that a solid object (tablet e.g.) is more easily handled than a powder when preparing a paste and, by facilitating the use of a solid object, body fluids may be used as the "solvent".

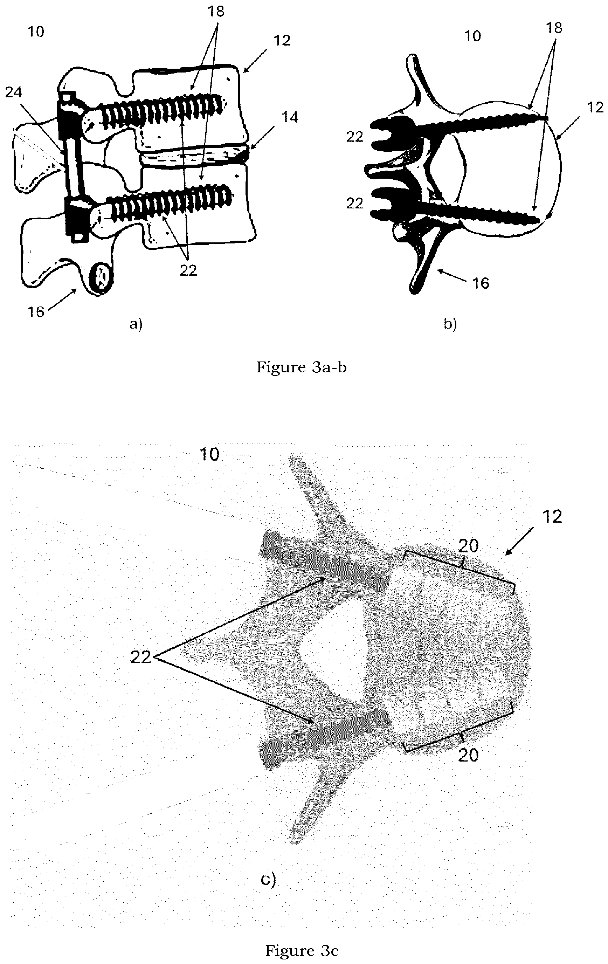

A particular advantage in clinical application of biomaterials is that the solid body enables accurate delivery and containment. Calcium based cement pastes that are injected are known to risk unwanted and potentially dangerous migration away from the target site. This is a known risk particularly in spinal surgery procedures. Instead, the present invention makes it possible to use a solid object adapted to fit into a cavity or void whereafter an aqueous solution such as body fluid is added to or allowed to soak the solid object to form the paste. Figure 3 illustrates how the solid body application reduces this risk.

A solid object according to the present invention has a density in the range of 1.20-1.80 g/cm³, preferably 1.30-1.70 g/cm³, preferably 1.40-1.65 g/cm³, more preferably 1.45-1.60 g/cm³. This provides the best properties regarding handling, adsorption rate and adhesive strength.

Present inventors have found that compressing a powder at a pressure of 10 to 150MPa results in a solid object which allows fast absorption and even distribution within the object, and the shape of the object is preserved even after absorption. Depending on the diameter, the widest cross-sectional length or the surface area the pressure results in different densities and thereby different absorption properties. A solid object of the present invention should not be too compact or too dense in order to allow fast and evenly distributed absorption, but should not be too loosely compressed because then the object will be hard to handle and would disintegrate too fast upon absorption. When compressing the powder at 10-150Mpa a solid object having sufficient mechanical properties and the absorption time is improved. For a solid object having a diameter or widest cross-sectional length of about 2-4mm or a surface area of 3-12mm² a pressure of 110-150Mpa is preferably used, for a solid object having a diameter or widest cross-sectional length of about 3.5-5.5mm or a surface area of 9.5-24mm² a pressure of 80-120Mpa is preferably used, for a solid object having a diameter or widest cross-sectional length of about 5 to 8mm or a surface area of 19.5-50mm² a pressure of 40-90Mpa is preferably used and for a solid object having a diameter or widest cross-sectional length of 8mm or more or a surface area of 50mm² or larger a pressure of 10-50Mpa is preferably used.

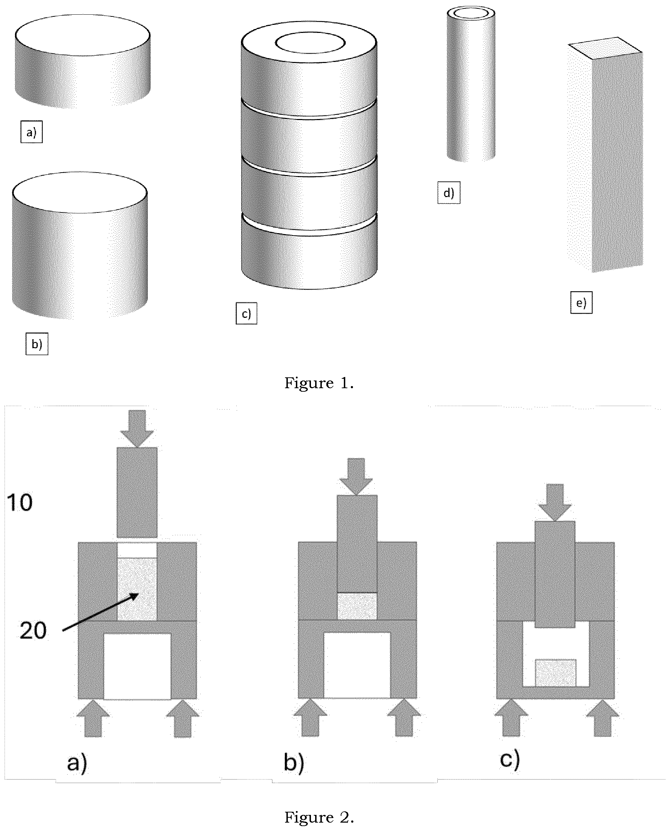

The height of the solid objects is preferably 1 mm or more, more preferably 2 mm or more, more preferably 3 mm or more, more preferably 4 mm or more, but preferably not more than 30 mm, preferably not more than 20mm, preferably not more than 10mm, preferably 8 mm or less, more preferably 7 mm or less, more preferably 6 mm or less. The diameter to height ratio is preferably 1-10, more preferably 1-3 providing good handling properties and allows reproducible results when preparing the solid objects. Figure 1 illustrates various solid objects according to the present invention having different shapes and ratios.

In order for the solid body to have the beneficial solubility and mixing property the particles of the powder should preferably have a mean particle size (D50) in a range of 1 to 20 µm, preferably 3-15 µm. The particle size of the powder has a distribution which may affect the properties of the solid object in various ways. A D10 value of 1 to 4.5µm of the particle size distribution is preferred. A D90 value of 30 to 450µm of the particle size distribution is preferred. Span or Dspan is a measure of the distribution of the particle size and is defined as (D90-D 10) / D50, where a value close to 2 means a uniform size distribution. The powder of the present invention preferably has a Dspan of 7 or less, preferably 3 or less, more preferably about 2. A lower Dspan results in solid objects with higher absorption rate.

The powder composition comprises a calcium phosphate compound, phosphoserine or phosphocreatine and preferably a calcium silicate. The calcium phosphate compound is preferably selected from tetra calcium phosphate (TTCP), α-tricalcium phosphate (α-TCP), β-tricalcium phosphate (β-TCP) or mixture thereof. In a preferred embodiment the calcium phosphate compound is α-TCP. Said compound in combination with phosphoserine and calcium silicate is believed to result in the best adhesive properties. The amount of the calcium phosphate compound in the powder is preferably 65-85 wt% based on the total weight of the solid object.

Without being bound by theory but it is believed that the phosphoserine or phosphocreatine acts as a curing agent providing improved mechanical strength to the composition. The amount of phosphoserine or phosphocreatine in the powder is preferably 15 to 35wt% based on the total weight of the solid object.

Calcium silicate is preferably a mono-, di- or tricalcium silicate or a mixture of two or more thereof. In one embodiment the calcium silicate is a mixture of di- and tricalcium silicate. The silicon compound may be an oxide such as [SiO₄]²⁻ or [Si₂O₇]⁶⁻ or quartz, feldspars, zeolites, micas, pyroxene. In one embodiment the silicate is selected from calcium silicate, sodium silicate and aluminum silicate, magnesium silicate, strontium silicate; zirconium silicate; or a mixture of di- and tri-calcium silicate preferably calcium silicate. The silicates may be in the form of a cement such as Portland grey cement or Portland white cement. A mixture of di- and tricalcium silicate may comprise between 10-90wt% of dicalcium silicate and between 10-90wt% of tricalcium silicate such as 30-70wt% of di-calcium silicate and 30-70wt% of tri-calcium silicate. The monocalcium silicate is preferably calcium metasilicate or wollastonite or pseudowollastonite. Using a calcium silicate increases the strength of the cured material.

In one embodiment the amount of the calcium phosphate compound in the powder is preferably 65-85 wt%, the amount of the phosphoserine is preferably 15-35 wt% and the amount of the calcium silicate is preferably 0.2-2 wt% or preferably 0.5-1.5wt%, all amounts are based on the total weight of the solid object.

The solid object may have any suitable geometric shape dependent on the application but preferably in the shape of a tablet, a ring, a sphere, a disc, a cylinder, a tube, a rectangular or cubic block, sheet or a cone. A cylinder or tube allows the insertion of a screw or an implant into the hollow part of the solid object. A block or sheet allows placement between opposing surfaces of a bony fracture.

An advantage of the present invention is that it makes it easier for the practitioner to use the powder composition and, by using a premade "plug" e.g. in form of a cylinder or a tube, that may be arranged at the site of operation the composition will interact quickly with the surrounding tissue by using body fluids to 'mix'. Further, this minimises "dead" material left in a syringe after using the injectable form, and provides a secured and immediate adhesion to the tissue and/or the screw or implant.

### Method of preparing solid object

When preparing the present solid object, a powder comprising a calcium phosphate compound, phosphoserine or phosphocreatine and preferably a calcium silicate is provided. To achieve the required particle size or particle size distribution the powder may be milled and or sieved. The powder is arranged in a mould configured to result in the desired shape of the solid object.

Referring to Figure 2. A die tool (10) may be used or any other suitable tool. The powder (20) is arranged in the mould of the tool (10) (Figure 2a). In order to prepare the solid object of the present invention a powder of the present invention is exposed to a pressure of 10 to 150Mpa for a predetermined period of pressing time. The pressing time is at least partly dependent on the size of the object but is generally at least 5 seconds or longer, preferably between 10 seconds and 60 seconds (Figure 2b). As disclosed herein the pressure used is dependent on the diameter or the cross-sectional length of the object. The pressing time is also partly dependent on the height or thickness of the final solid object. The solid object is then removed from the mould and is preferably stored in a sealed container or bag in order to minimize moisture absorption (Figure 2c). The sealed container or bag is preferably filled with an inert gas such as nitrogen or argon.

### Method of treatment

A particular advantage in clinical application of biomaterials is that the present invention enables accurate delivery and containment. Calcium based cement pastes that are injected are known to risk unwanted and potentially dangerous migration away from the target site. This is a known risk particularly in spinal surgery procedures.

The present solid object may be used for treating a patient by arranging the solid object in a cavity or a void in a bone. Aqueous solution e.g. body fluid is allowed to be absorbed by the solid object optionally together with added aqueous solution. The formed paste is left to cure to form a cured body. Limited about of body fluid or aqueous solution is needed to form the paste and therefore migration of the potentially toxic calcium phosphate is limited or even avoided.

Turning to Figure 3a-c. When treating a spinal injury, it is important to fixate the injury without migration or leakage of the calcium cement. The spine (10) comprises a vertebral body (12), intervertebral disk (14) and lower articular process (16). In an illustrative treatment of an injury or defect in a spine (10) two or more holes (18) are prepared in two adjacent vertebral bodies (12) preferably by drilling and the size of the holes are such that a solid object (20) according to the present invention may fit into each hole (18). In one embodiment at least two holes are prepared in at least two adjacent vertebrate bodies. The solid object (20) according to the present invention, preferably in the form of a tube, ring, a cylinder or a stack of rings, is arranged in each hole (18). In Figure 3c the solid object (20) is illustrated as comprising a stack of four rings. Body fluid and optionally also an aqueous solution is allowed to be absorbed by the solid object forming a paste. Before the paste has cured a screw (22) is inserted in each solid object (20) and then the paste is left to cure. Preferably the screws (22) are pedicle screws. The addition of the screw ensures the paste formed from the solid object is pressed into the surrounding tissues for optimal fixation of the screws. Screws (22) are connected by a rod (24) in order to further fixate the spine (10).

When forming the paste from the solid object an aqueous solution is added to the solid object. Liquid (aqueous solution) to powder ratio (L/P) determines the viscosity and the paste handling properties of the aqueous composition and is an important factor when it comes to curing time. In order to obtain suitable viscosity and paste handling properties as well as appropriate curing time the L/P is preferably 0.1-0.50, more preferably 0.2-0.40, and more preferably about 0.3. Which is less than what could be expected.

### Kit

The kit according to the present invention comprises at least one solid object according to the present invention and a syringe. The syringe comprises a chamber and a plunger arranged in the chamber wherein the syringe is configured to receive the solid object and an aqueous solution. When pushing the plunger the aqueous solution is mixed with the solid object and pushes the formed paste through the nozzle of the syringe.

### EXAMPLES

### Example 1

Powder components were prepared using the following formulation:
- aTCP 74.5wt% (Particle size: D10 - 1.16µm, D50 - 5.80µm, D90 - 37.11µm, Dspan - 6.2)
- Phosphoserine - 24wt%
- Calcium Silicate - 1.5wt%

Tablets were prepared by placing 0.171g (Sample 1) and 0.207g (Sample 2) of the powder into a 6mm diameter steel die tool and compressed at a load of 86MPa for 60 seconds. This resulted in tablets with approximately 4 and 5mm height respectively and with a density of approximately 1.52 and 1.46g/cm³ respectively (mean density of 1.49g/cm³).

Several sample tablets were prepared using the same method resulting in similar densities.

### Compressing testing

0.06ml of type 1 filtered water (ultrapure) was pipetted onto a weigh boat. Sample 2 was placed into the droplet of water which was rapidly absorbed into the tablet. The tablet was left to cure for 60 minutes in a water bath at 37°C. The cured sample was then tested using Zwick compression tester. Peak result was 19.85Mpa. For comparison cancellous bone compression strength is approx. 10MPa.

### Torque testing

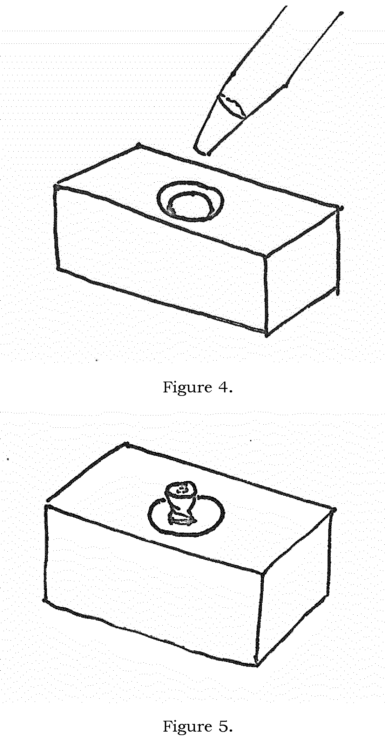

One tablet was placed into a prepared socket of 6.5mm diameter by 8mm deep in a Sawbones block. 0.08ml of Type 1 filtered water was pipetted into the socket on top of the tablet (Figure 4). A second and third tablet were then placed on top with water added onto each tablet in order. A 3.3mm diameter x 10m long Titanium dental implant was placed into the socket through the formed paste (Figure 5). The sample was left to cure in a temperature and humidity-controlled box that mimics conditions in the mouth (31°C, 90% humidity) for 20 minutes.

The torque resistance of the implant was measured using a torque driver within the test box. The peak torque achieved was 39.1Ncm. Dental implants are deemed stable at a torque in value of 35Ncm.

Repeating the experiment with 34% Phosphoserine content increased the peak torque out to 49.6Ncm at 1 hour.

### Example 2

Powder components were prepared using the following formulation:
- aTCP 75.25wt% (Particle size: D10 - 3.6µm, D50 - 12.4µm, D90 - 35.0µm, Dspan - 2.53)
- Phosphoserine - 24wt%
- Calcium Silicate - 0.75wt%

0.207g of mixed powder was placed into a 6mm diameter steel die tool and compressed at a load of 86MPa for 60 seconds. This results in a tablet approximately 5mm high with a density of approximately 1.46g/cm³.

Several sample tablets were prepared using the same method resulting in similar densities.

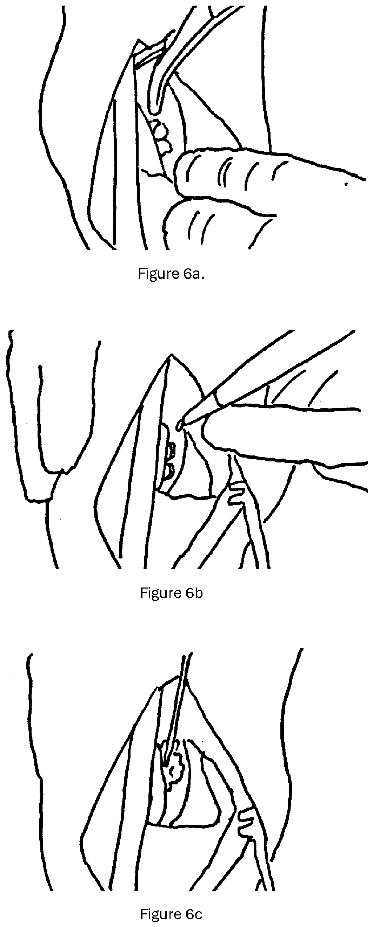

The talus bone in an adult male fresh frozen cadaveric specimen was resected using a bone saw in the sagittal plane approximately 10mm from the lateral side to mimic a talus fracture. Three tablets, diameter 6mm, 5mm height were placed into the space between the joint surfaces (Figure 6a). 1ml of water was added over the tablets (Figure 6b). After a few seconds the tablets turned to a paste and were spread over the surfaces of the bone using a K-wire (Figure 6c). The bones were closed together and 2 K-wires were drilled through the bony construct to hold the fragment in place. The K-wires remained in place for 40 minutes.

Upon removal of the K-wires, the bone fragment remained in place and allowed full movement of the ankle joint without failing. A handheld force gauge was then used to apply a shear force to the medial fragment of the talus. A force of 193N was reached without the bone fragments separating. Loading to higher forces was not possible using this method.

### Example 3

Powder components were prepared using the following formulation:
- aTCP 75.25wt% (Particle size: D10 - 3.6µm, D50 - 12.4µm, D90 - 35.0µm, Dspan - 2.53)
- Phosphoserine - 24wt%
- Calcium Silicate - 0.75wt%

Samples were prepared by placing for example 0.067, 0.053 and 0.067g powder into a 3mm diameter steel die tool and compressed at a load of 141MPa for 60 seconds. This resulted in tablets having a height of approximately 6, 4.8 and 6mm respectively and having a density of approximately 1.58, 1.56 and 1.57g/cm3 respectively (mean density of 1.57g/cm³).

Several sample tablets were prepared using the same method resulting in similar densities.

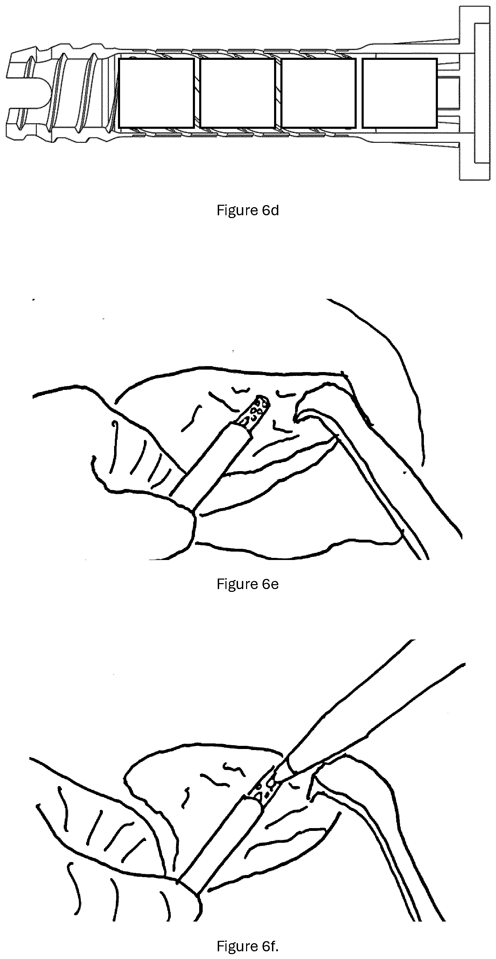

Four tablets were placed inside a prototype metal screw reinforcement implant (Figure 6d) comprising a tube with fenestrations and openings to allow the paste to pass through into the bone. A screw is threaded into the implant for fixation into the bone. A wax plug was used to seal the end of the tube to hold the solid dose in place.

A hole was pre-drilled into the Proximal Humerus of an adult male fresh frozen cadaveric specimen. The implant pre-loaded with solid objects was partially placed into the hole (Figure 6e).

1ml of water was pipetted over the solid dose components and the implant was fully pushed into the bone (Figure 6f).

A cortical bone screw was fully threaded into the implant. This has the effect of spreading and pressurizing the premixed paste into the surrounding bone.

After 40 minutes the implant was pulled by hand from the bone. A force of 169N was achieved. In comparison, pull-out forces of a screw in native bone without adhesive have been measured in the region of 50N.

### Example 4

Powder components were prepared using the following formulation:
- aTCP 75.25wt% (Particle size: D10 - 3.6µm, D50 - 12.4µm, D90 - 35.0µm, Dspan - 2.53)
- Phosphoserine - 24wt%
- Calcium Silicate - 0.75wt%

Two samples were prepared by placing 0.294 and 0.293g of the powder into a 8 mm diameter steel die tool and compressed at a load of 48MPa for 60 seconds. This resulted in tablets having a height of approximately 4 mm and having a density of 1.46 and 1.45cm3 respectively.

## Claims

1. A solid object having a shape wherein the solid object comprises a powder wherein the powder is a mixture of particles wherein said particles comprises a calcium phosphate compound and phosphoserine or phosphocreatine; and wherein the solid object has a density of 1.20-1.80g/cm³.

2. The solid object according to claim 1 wherein the density is 1.30-1.70 g/cm³, preferably 1.40-1.65 g/cm³, more preferably 1.45-1.60 g/cm³.

3. The solid object according to any one of the preceding claims wherein the shape is a tablet, a ring, a sphere, a disc, a cylinder, a tube, a rectangular or cubic block, sheet or a cone.

4. The solid object according to any one of claim 1 to 3 wherein the height of the solid object is 1 mm or more, preferably 2 mm or more, more preferably 3 mm or more, more preferably 4 mm or more, but preferably not more than 10 mm, preferably 8 mm or less, more preferably 7 mm or less, more preferably 6 mm or less.

5. The solid object according to any one of claim 1 to 4 wherein the particles have a mean particle size of 1 µm to 20 µm, preferably 3-15µm.

6. The solid object according to any one of claim 1 to 5 wherein the particles have a Dspan of 7 or lower, preferably 3 or lower, but preferably higher than 2.

7. The solid object according to any one of the preceding claims wherein the calcium phosphate compound is selected from tetra calcium phosphate (TTCP) or α-tricalcium phosphate (α-TCP).

8. The solid object according to any one of the preceding claims wherein the amount of calcium phosphate is 65-85wt%; wherein the amount of phosphoserine or phosphocreatine preferably is 15 to 35wt% of the total weight of the solid object, preferably 20 to 30wt%, more preferably 22 to 27wt%; and wherein the powder preferably comprises calcium silicate in an amount of 0.2-2wt%, preferably 0.5-1.5wt% wherein the calcium silicate is preferably selected from a mono-, di- or tricalcium silicate or a mixture of di- and tricalcium silicate.

9. A method of producing the solid object according to any one of claim 1 to 8 wherein the method comprises the steps of:
a. Providing a powder mixture comprising a calcium phosphate compound, phosphoserine and optionally a calcium silicate;
b. Arranging the powder in a mould having the shape; and
c. Compressing the powder mixture during a first period of time at a pressure of 10-150MPa.

10. The method according to claim 9 wherein the pressure is 110-150MPa when the solid object has a diameter of about 2-4mm or a surface area of 3-12mm².

11. The method according to claim 9 wherein the pressure is 80-120MPa when the solid object has a diameter of about 3.5-5.5mm or a surface area of 9.5-24mm².

12. The method according to claim 9 wherein the pressure is 40-90MPa when the solid object has a diameter of about 5-8mm or a surface area of 19.5-50mm².

13. The method according to claim 9 wherein the pressure is 10-50MPa when the solid object has a diameter of about 8mm or larger or a surface area of 50mm² or larger.

14. The method according to claim 9 wherein the first period of time is at least 10 seconds, preferably 30 to 120 seconds.

15. A kit comprising at least one solid object according to any one of claim 1 to 8 and a syringe comprising a chamber and a plunger arranged in the chamber; wherein the syringe is configured to receive the solid object and an aqueous solution, and wherein the syringe is further configured to mix the aqueous solution with the solid object when pushing the plunger.
